# EUROPEAN PATENT APPLICATION

(11) **EP 0 629 393 A2**
(43) Date of publication of application: **21.12.1994**
(21) Application number: 94304083.2
(22) Date of filing: 07.06.1994
(51) Int. Cl.: A61K 7/00

(54) **A crystalline polyol composition**

(30) Priority: 16.06.1993 US 78706
(71) Applicant: ICI AMERICAS Inc., Wilmington, Delaware 19859 (US)
(72) Inventor: Duross, James Wix, Smyrna, DE 19977 (US)
(74) Representative: Thomas, Ieuan

(57) **Abstract**

A crystalline polyol composition having improved cariogenic properties derived from at least one mono- or polysaccharide having at least one phosphopeptide compound or salt thereof uniformly dispersed within its crystal structure is disclosed. Also disclosed is a method of producing such a crystalline polyol composition by the controlled crystallization of the molten sugar alcohol having the phosphopeptide compound or salt thereof dispersed therein.

## Description

### FIELD OF THE INVENTION

This invention relates to crystalline polyol compositions having improved cariogenic properties comprising at least one crystalline sugar alcohol, having uniformly dispersed within its crystalline structure at least one phosphopeptide compound or salt thereof, and to a method of producing such crystalline polyol compositions.

### BACKGROUND OF THE INVENTION

It is known that some phosphopeptide compounds (the term phosphopeptide compounds is meant to include phosphopeptide salts) are useful for inhibiting caries and gingivitis.

It is also known that polyols, in particular sorbitol, mannitol, and xylitol have been utilized in a variety of food-confectionery-pharmaceutical formulations which have been marketed to the consumer as products, which promote dental hygiene through the reduction of dental caries.

To achieve maximum benefit from the incorporation of the phosphopeptides into food-confectionery products, oral pH should be maintained as close to neutral as possible and above a pH of 5 for any benefit at all. Maintenance of a neutral pH is difficult to achieve in food-confectionery and pharmaceutical systems where "conventional sugars" constitute a large percentage of the "finished formulation". Oral bacteria utilize the carbohydrates as a source of energy and excrete lactic acid, lowering oral pH resulting in demineralization of tooth enamel with subsequent tooth decay. The rate of demineralization of the enamel is directly related to the amount of time the dental enamel is exposed to low oral pH (<5.5). Traditionally complex and difficult buffering of sucrose-based food-confectionery products was done to minimize the drop in oral pH during consumption of these food-confectionery and pharmaceutical products. A reduction in dental caries has been achieved by substituting sugar alcohols for the conventional sugars.

The reduction in dental caries is achieved by virtue of the fact that the sugar alcohols are not metabolized by the common microflora found in the oral cavity (s. mutans) which results in very little acid generation to lower the oral pH. pH telemetry studies have shown that the use of sorbitol, mannitol, xylitol, or combinations thereof in sucrose-free gum, tablet, or candy formulation results in oral pH maintenance in the 5.8 to 7.1 range, the optimum range for maximum control of calcium deposition from saturated oral solutions of calcium phosphate. Thus, a crystallized polyol composition containing one or more selected polyols and at least one phosphopeptide would provide the formulator, particularly of sucrose-free products, with a novel composition having the synergy of two products to enhance dental hygiene, one maintaining oral pH, the other a source of calcium ions to promote/enhance remineralization.

Thus, it would be desirable to possess a crystalline polyol composition which contains a uniform concentration of at least one phosphopeptide compound which composition can be easily and inexpensively manufactured with low risk of contamination. Accordingly, it is an object of this invention to provide a crystalline polyol composition having a uniform dispersion of phosphopeptide compounds or salts thereof having improved cariogenic properties.

It is a further object of this invention to provide crystalline polyol compositions that can be ground to a fine powder and still maintain a uniform distribution of the phosphopeptide compounds.

Another object of this invention is to provide crystalline polyol compositions providing a controlled release of the phosphopeptide compound over a longer time frame which will cause the formation of a metastable solution of calcium phosphate resulting in deposition of calcium ions from this solution on the dental enamel.

It is still another object of this invention to provide crystalline polyol compositions suitable for formulating into food confectionary products and pharmaceutical products.

It is a further object of this invention to provide a process for economically preparing crystalline polyol compositions having a uniform dispersion of at least one phosphopeptide compound.

The above objects and other additional objects will become more fully apparent from the following description and accompanying Examples.

### SUMMARY OF THE INVENTION

In one aspect, the present invention is directed to crystalline polyol compositions comprising at least one crystalline sugar alcohol derived from a mono- or polysaccharide having at least one phosphopeptide compound uniformly dispersed within its crystalline matrix.

In another aspect, this invention is directed to a process for producing crystalline polyol compositions having at least one phosphopeptide compound uniformly dispersed therein, said process comprising the steps of:
(A) forming a molten sugar alcohol derived from at least one mono- or polysaccharide;
(B) dispersing at least one phosphopeptide compound in said molten sugar alcohol under conditions such that a homogeneous mixture is formed;
(C) cooling said homogeneous molten mixture under agitation until a viscous mass is formed: and
(D) cooling said mass slowly until the sugar alcohol becomes fully crystallized.

### DESCRIPTION OF THE PREFERRED EMBODIMENTS

The crystalline polyol compositions of this invention are comprised of at least one crystalline sugar alcohol derived from a mono- or polysaccharide having at least one phosphopeptide compound uniformly dispersed within its crystal matrix.

The sugar alcohols which may be employed in the practice of this invention are well known to those of skill in the art and are typically produced by the catalytic hydrogenation of mono- and/or polysaccharides derived from carbohydrates which are high molecular weight polymers derived from pentose and hexose units. Illustrative of such saccharide materials are sugars; such as dextrose and maltose; cellulose, starch, and wood sugars. These materials are typically hydrolyzed under aqueous conditions utilizing enzymes or mineral acids to form monoses, dioses and trioses, etc. which are then catalytically reduced with hydrogen by well known methods. The aqueous solutions of the sugar alcohols formed thereby are then typically treated with ion exchange resins, activated carbon, or the like to form clear solutions.

Illustrative of the sugar alcohols which may be employed in the practice of this invention are mannitol, sorbitol, cellobiitol, lactitol, xylitol or blends thereof. The preferred sugar alcohols are sorbitol, xylitol, mannitol and blends thereof. The most preferred sugar alcohols are sorbitol, blends of sorbitol/mannitol and blends of sorbitol/xylitol. Where a blend of sorbitol/mannitol is employed in the composition the sorbitol is typically present in an amount ranging from 70 to 99 percent by weight, based on the total weight of the sugar alcohol blend, preferably in an amount ranging from 85 to 95 percent by weight. The mannitol is typically present in an amount ranging from 1 to 30 percent, preferably 5 to 15 percent by weight based on the total weight of the sugar alcohol blend. Where a blend of sorbitol/xylitol are employed in the composition the sorbitol is typically present in an amount ranging from 65 to 95 percent, by weight based on the total weight of the sugar alcohol blend, preferably in an amount ranging from 70 to 75 percent by weight. The xylitol is typically present in an amount ranging from 5 to 35 percent, preferably 25 to 30 percent, by weight based on the total weight of the sugar alcohol blend.

The phosphopeptides of the present invention or their salts may have utility in the treatment or inhibition of dental diseases such as caries, gingivitis and periodontal disease. Illustrative of the phosphopeptides are those disclosed in U.S. Patent 5,015,628 (granted May 14, 1991; Reynolds), hereby incorporated by reference. In particular, the phosphopeptides or salts thereof have from 5 to 30 amino acids including the sequence
A-B-C-D-E
wherein A, B, C, D and E are independently phosphoserine, phosphothreonine, phosphotyrosine, phosphohistidine, glutamate and aspartate. Preferred phosphopeptides are those wherein A, B, and C are independently phosphoserine, phosphothreonine, phosphotyrosine and phosphohistidine and D and E are independently phosphoserine, phosphothreonine, glutamate and aspartate. Particularly preferred phosphopeptides are those wherein A, B and C are phosphoserine and D and E are glutamate.

In general, such phosphopeptides can be produced by a tryptic digestion of bovine milk casein (as disclosed in U.S. Patent 5,015,628, Reynolds). The digestion of whole sodium caseinate or fractions (alpha-S or beta) produced by selective precipitation (Zittle, C. A. and Custer J. H.; J. Dairy Sci 46L 1183-1189, 1963) is carried out using a protein:trypsin ratio of 50:1 in 20mM Tris HCl pH 8.0, 2.5 mM NaCl at 37°C for 1 hr. The peptides are fractionated using a Pharmacia FPLC system with a Mono Q HR 5/5 column and eluted with a NaCl gradient; Buffer A 10 mM Tris HCl pH 8.0, 2.5 mM NaCl; Buffer B 20 mM Tris HCl pH 8.0, 500 mM NaCl, gradient 0-100%; Buffer B/30 min; flow rate 1 ml/min. Fractions are washed and concentrated using an Amicon Ultrafiltration Cell with a UMOS filter. The peptides are identified using a Water Associates PICO-TAG amino acid analysis system using phenylisothiocyanate amino acid derivatization. Phosphate is measured by the method of Itaya and Ul (Clin. Chem, Acts 14:361-366, 1960). The peptides are sequenced (after the removal of phosphate by alkaline phosphatase) using manual Edman degradation and the resulting PTH-amino acids identified using reverse phase HPLC on a Zorbax ODS column 25x0.46 cm (DuPont).

The following phosphopeptides individually obtained from a tryptic digestion of sodium caseinate using the above procedure may be employed in the present invention:
T1. Glu-Met-Glu-Ala-Glu-Pse-Ile-Pse-Pse-Pse-Glu-Glu-Ile-Val-Pro-Asn-Pse-Val-Glu-Gln-Lys.
T2. Glu-Leu-Glu-Glu-Leu-Asn-Val-Pro-Gly-Glu-Ile-Val-Glu-Pse-Leu-Pse-Pse-Pse-Glu-Glu-Ser-Ile-Thr-Arg.
T3. Asn-Thr-Met-Glu-His-Val-Pse-Pse-Pse-Glu-Glu-Ser-Ile-Ile-Pse-Gln-Glu-Thr-Tyr-Lys.
T4. Asn-Ala-Asn-Glu-Glu-Glu-Tyr-Ser-Ile-Gly-Pse-Pse-Pse-Glu-Glu-Pse-Ala-Glu-Val-Ala-Thr-Glu-Glu-Val-Lys.
T5. Glu-Gln-Leu-Pse-Pth-Pse-Glu-Glu-Asn-Ser-Lys.

The peptide T1, is also obtained from a TPCK tryptic digest of alphaₛ₁ -caseinate (comprising alphaₛ₁ and alphaₛ₀). Peptide T2 is also obtained from a TPCK-tryptic digest of beta-caseinate. Peptides T3, T4, and T5, are also obtained from a TPCK- tryptic digest of alphaₛ₂ caseinate (comprising alphaₛ₂, alphaₛ₃, alphaₛ₄, and alphaₛ₆). The amino acid symbols are as follows: Pse-phosphoserine, Ser-serine, Pth-phosphothreonine, Thr-threonine, Glu-Glutamate, Asp-aspartate, Ala-alanine, Asn-aspargine, Glu-glutamine, Gly-glycine, Arg-arginine, His-histidine, Ile-isoleucine, Leu-leucine, Lys-lysine, Met-methionine, Pro-proline, Tyr-tyrosine, Val-valine.

The phosphopeptide useful in this invention can also be produced by the following procedure.

A solution of sodium caseinate is digested with trypsin (50:1, casein:trypsin) for one hour at 37°C. with the pH maintained at 8.0 by the addition of NaOH, HCl (0.1M) is then added to the solution at room temperature to pH 4.7 and the resulting precipitate removed. BaCl₂ is added to the supernatant to a level of 0.25% w/v followed by an equal volume of absolute ethanol and the resulting precipitate is removed and dried. The precipitate is dissolved in one tenth the original volume of water (to facilitate dissolution the pH is raised with NaOH) and the solution acidified to pH 3.5 with 1M HCl. An equal volume of acetone is added and the precipitate is removed and dried. The precipitate is then redissolved in H₂O and acidified to pH 2.0 by addition of HCl. The resulting precipitate is removed and discarded and the supernatant is adjusted back to pH 3.5 with NaOH and an equal volume of acetone is added. The resulting precipitate was collected, redissolved in water and H₂SO₄ added to precipitate BaSO₄ which is discarded. The supernatant is then dialyzed and lyophilized or spray dried. A mixture of 5 phosphopeptides are obtained with this procedure.

The following are the phosphopeptides obtained:
T1. Glu-Met-Glu-Ala-Glu-Pse-Ile-Pse-Pse-Pse-Glu-Glu-Ile-Val-Pro-Asn-Pse-Val-Glu-Gln-Lys.
T2. Glu-Leu-Glu-Glu-Leu-Asn-Val-Pro-Gly-Glu-Ile-Val-Glu-Pse-Leu-Pse-Pse-Pse-Glu-Glu-Ser-Ile-Thr-Arg.
T3. Asn-Thr-Met-Glu-His-Val-Pse-Pse-Pse-Glu-Glu-Ser-Ile-Ile-Pse-Gln-Glu-Thr-Tyr-Lys.
T4. Asn-Ala-Asn-Glu-Glu-Glu-Tyr-Ser-Ile-Gly-Pse-Pse-Pse-Glu-Glu-Pse-Ala-Glu-Val-Ala-Thr-Glu-Glu-Val-Lys.
T5. Glu-Gln-Leu-Pse-Pth-Pse-Glu-Asn-Ser-Lys.

The ratio of the phosphopeptides (T1:T2:T3:T4:T5) in the final preparation depends on the starting material and conditions of hydrolysis. Digesting sodium caseinate with TPCK-trypsin yields largely T2 with small amounts of T1, T3 and T4. However, T2 shows greater ability than the other peptides such that more rigorous digestion as occurs with some commercial casein digests yields a preparation containing largely T1 with small amounts of T3 and T4.

If in lieu of sodium caseinate, alpha s₁-casein is used for this procedure pure T1 is obtained. With beta-casein as the starting material pure T2 is obtained.

The most common sequences of the active peptides is the pentapeptide Pse-Pse-Pse-Glu-Glu.

Conservative substitutions within the active sequence would be phosphothreonine and to a lesser extent phosphotryrosine or phosphohistidine for phosphoserine although phosphoserine is preferable. Another possible substitution for phosphoserine would be glutamate or aspartate but again phosphoserine is preferable. A possible substitution for glutamate is aspartate.

The active peptides can sequester calcium phosphate and other salts of divalent metal ions. One mole of T1 binds 16 moles of CaHPO₄ such that a 10mg/ml solution of T1 at pH 7.0 can solubilize 60 mM CaHPO₄ producing a metastable supersaturated solution with respect to calcium phosphate species. With chloride as the counter ion one mole of T1 binds only 5 moles of Ca++ binding only to serine phosphates. One mole of T1 with about 16 moles of CaHPO₄ bound (M.W. 4883) will henceforth be referred to as calcium phosphate T1. An important chemical feature of calcium phosphate T1 is that above 2% w/v in water the composition is a thixotropic gel.

The sugar alcohols employed herein generally are dried such that they have a water content of less than about 3 percent by weight. Preferably such water content is less than about 1 percent and most preferably is less than about 0.5 percent by weight. The sugar alcohol starting materials may be dried to the desired water content by conventional means such as a continuous thin film evaporator or a batch vacuum cooker.

It is possible to uniformly formulate up to about 30 percent or more by weight based on total weight of one composition of the phosphopeptide compound in the polyol composition following the process of this invention. However, the phosphopeptide compound is more typically present in an amount less the 18 percent, preferably less than 12 percent.

The crystalline polyol compositions of this invention are prepared by:
(A) forming a molten sugar alcohol derived from at least one mono- or polysaccharide;
(B) dispersing at least one phosphopeptide compound in said molten sugar alcohol under conditions such that a homogeneous mixture is formed;
(C) cooling said homogeneous molten mixture under agitation until a viscous mass is formed, and
(D) cooling said mass slowly until the sugar alcohol becomes fully crystallized.

In step (A), one of ordinary skill in the art can easily determine suitable operating temperatures by routine experimentation, typical operating temperature ranges for the following exemplary sugar alcohols are as follows; sorbitol, between about 86° and about 130°C; xylitol, between about 80° and about 120°C; mannitol, between about 140° and about 170°C; lactitol, between about 100° and about 200°C; cellobiitol, between about 100° and about 175°C and hydrogenated starch hydrolysate between about 150° and about 200°C. It should be noted, however, that the deactivation temperature of the phosphopeptide compounds must be taken into account when selecting the appropriate sugar alcohol to be employed, as well as the specific temperature within the acceptable operating temperature range for a given sugar alcohol.

In step (B) of the process hereof the phosphopeptide compounds are dispersed in the molten sugar alcohol under conditions such that a homogeneous mixture is formed. The phosphopeptide compound is added directly to the molten alcohol using any conventional addition technique. Care must be taken, however, to ensure that agitation continues at the elevated temperature of the molten alcohol until thorough dispersion of the active compound in the molten sugar alcohol has occurred.

Optionally, steps (A) and (B) can be reversed such that the molten sugar alcohol is added to the phosphopeptide compounds or a further option is that the phosphopeptide compounds and the sugar alcohol can be mixed prior to heating and then formed into a homogeneous molten mixture.

A further option is to form the molten sugar alcohol of step (A) then remove an aliquot of the molten sugar alcohol, in an amount at least equivalent by weight to the amount of phosphopeptide compound being added, mix the phosphopeptide compound into the molten sugar alcohol, by any conventional mixing technique, until the phosphopeptide is thoroughly dispersed. Then reintroduce the removed aliquot of phosphopeptide/sugar alcohol into the sugar alcohol molten mass as outlined in step (B), above.

Once the phosphopeptide compound has been uniformly dispersed in the molten alcohol (which point can be readily determined by routine assay for any particular additive/molten alcohol combination) the temperature is reduced while agitation continues. Such cooling with agitation results in the onset of crystallization. Agitation should be continued until the formulation becomes a viscous mass. By the term "viscous mass" is meant a composition which has a semi-solid dough-like appearance, is extrudable and is not liquid and runny. Typically, at this point the sugar alcohol is generally at least about 40 percent crystalline by weight. However, where high loadings of non-crystalline polyol ingredients are present, a viscous mass may be present where as little as only 20 weight percent of the sugar alcohol need be crystallized. If desired, the dispersion may be periodically monitored e.g., by differential scanning calorimetry or other reliable methods known in the industry, until the required percentage crystallinity for a given sugar alcohol phosphopeptide mixture (which percentage can easily be determined by running trials at various times until a suitable viscous mass is formed and then determining the crystallinity of such viscous mass, e.g. by differential scanning calorimetry) is observed.

The viscous mass is removed from the agitating means and allowed to further cool until a solid crystalline mass having a uniform dispersion of phosphopeptide compound is obtained. While the mixture can fully crystallize under agitation, this is generally not preferred.

The fully crystalline mass may be ground, employing conventional grinding equipment to provide a powder which can be formed into an orally ingestible diluent (such as comestibles, foodstuffs and/or confections) and/or pharmaceutical excipients.

Of particular interest as compositions are chewing gum, breakfast foods, ice-cream and other frozen confectionery, confectionery, sweets and cakes as these are all known as caries problem materials.

The composition of this invention may be in the form of a comestible such as foodstuff or confectionery, dentifrice, tablet or comprise a pharmacologically acceptable vehicle or solution of suspension for topical application to the teeth or gingival tissues or a mouthwash.

Large scale preparations may preferably be made employing a process wherein the sugar alcohol preferably sorbitol or a blend of sorbitol and mannitol is heated to a temperature of between about 80°C and about 100°C and subjected to agitation in a heated tank. After addition of the phosphopeptide compound under continuous agitation the reaction mass is then metered into a continuous twin shaft mixer of the intermeshing type. Mixers of this type are discussed in "Chemical Engineers Handbook", 5th Edition, edited by R. H. Perry and C. H. Chilton (1973) pages 19-21. Characteristics of these mixers are that they include intermeshing kneader blades mounted on two parallel shafts which rotate in the same direction at the same speed with close blade-to-wall and blade-to-blade clearances. A key characteristic of these types of mixers is that they be jacketed to maintain adequate control over the sugar alcohol/phosphopeptide mixture during agitation.

A preferred continuous mixer is the high shear Readco Continuous Processor made by Teledyne Readco of York, Pa. The mixer shown in U.S. Pat. No. 3,419,250 and in U.S. Pat. No. 3,618,902 (both assigned to Teledyne Inc.) can be used without modification; however, the sugar alcohol magma which is formed in the present process is much more easily handled if the mixer is equipped with an extrusion nozzle or plate. Other high shear continuous twin screw mixers which impart a high shearing force at low shaft speed to the material being processed can also be used. Such mixers include the Baker-Perkins Multi-Purpose (M-P) mixer made by Baker-Perkins Inc. of Saginaw, Michigan and the ZSK Twin Screw Compounding Extruder made by Werner and Pfleiderer Corporation of Stuttgart, Germany, The Baker-Perkins mixer is shown in U.S. Pat. Nos. 3,195,868 and 3,198,491. Alternative blade configurations for mixers of this type are shown in U.S. Pat. No.s 3,423,074 (assigned to Baker-Perkins) and 3,490,750 (assigned to Teledyne, Inc.). These mixers are available in various diameters and horsepowers depending on the throughput required.

Preferably, a Readco Continuous Processor with kneader blade diameters of 2,5, 15 or 24 inches with feed and/or discharge screws is utilized. Further, the discharge nozzles are preferably provided with heating means in order that the surface of the partially solidified cylindrical ribbon of exiting magma does not prematurely crystallize ensuring a smooth discharge. Thus, one process for producing the crystalline polyol composition of this invention involves continuously introducing a feed comprising the molten magma containing the added phosphopeptide compound into an elongated mixing zone having shaft means and a plurality of kneader blades mounted on the shaft means, the configuration of the kneader blades being such as to provide restricted clearances between the blades and the adjacent walls; simultaneous cooling and kneading molten alcohol magma as it passes through the mixing zone until a viscous mass of molten sugar alcohol and phosphopeptide is obtained and continuously discharging the blend from the mixing zone through an extrusion orifice and further cooling the blend to ambient temperature forming the crystalline sugar alcohol comprising phosphopeptide compound.

In carrying out the crystallization, the molten sugar alcohol is preferably held in an agitated feed tank in a relatively dry atmosphere to inhibit moisture pickup such that the moisture content does not exceed about 1% by weight. This precaution becomes less of a factor as the temperature of the molten alcohol mix exceeds 100°C. At this point, the phosphopeptide compound is added under agitation (e.g. high shear mixing) blended with some of the crystalline polyol, or with melted polyol or melted and/or dispersed in molten polyol depending on the melt temperature of the polyols as well as on the specific physical characteristics of such phosphopeptide compound. In the operation of the mixing equipment, the feed rate and other operating parameters are adjusted such that as the cooling mass passes through the mixer, a molten blend having increased concentrations of crystals is generated as the magma passes through from the feed to the discharge orifice. The rotating screws move the molten magma containing crystals and dispersed phosphopeptide from the center of the equipment to the outer cooled edge whereupon additional crystals are formed and remixed with additional molten sugar alcohol and phosphopeptide to act as a crystallizing seed. As the temperature profile drops from molten feed temperature to discharge temperature, the viscosity of melt increases due to the formation of the crystals. The action of the rotating screws pushes the crystallizing molten magma containing dispersed ingredient in the form of extrudate through the discharge orifice whereupon it is extruded as an elongated mass. The extrudate may then be conveniently cut into desired lengths for grinding to the desired particle size distribution and permitted to cool until crystallization is complete.

Care should be taken to ensure that the temperature of the emitted extrudate is not too hot as the molten mass will lose its shape. Not only is such material difficult to handle, but the product obtained may be an undesirable mixture of crystals and amorphous sugar alcohol glass which, although still results in a product having a uniform dispersion of the phosphopeptide compound therein, is difficult to grind. The problem can be corrected by decreasing the throughput time or jacket cooling temperature and other variables such as feed temperature, rotation speed, back pressure, etc. Under ideal operating conditions the extrudate crystalline paste develops a solid outer shell of crystalline product which is only slightly wetter on the interior side with molten material. The hot extrudate when permitted to stand will fully crystallize as a function of time depending on the cross-sectional dimension of the extrudate mass (which typically ranges in cross-section from about 5 to about 20 millimeters) and the effect of the added phosphopeptide. Longer periods may be required for extruded shapes having a cross-sectional dimension of greater than 20 millimeters.

The extrudate may subsequently be formulated into various foods, confections and pharmaceuticals end use compositions such as tablets, chewing gum, toothpaste and the like using methods and formulations well known to those of skill in the art.

The following Examples are intended to further illustrate the invention and are not intended to limit the scope of the invention in any matter whatsoever. In such Examples all proportions expressed are by weight unless otherwise specified.

### Example 1

### Crystalline Sorbitol/Phosphopeptide

Into a 60 gallon steam heated vacuum cooker is pumped 400 lbs. of an aqueous solution of 50%, by weight, sorbitol. Under agitation a vacuum of 26-30 inches of Hg is pulled while gradually heating the sorbitol solution up to 120°C until no water is collected in the condenser. The resultant molten polyol should contain no more than 0.3% by weight water. Such molten polyol is then transferred to a heated Kettle equipped with a high shear mixer and maintained at 108°C while agitated. To this molten mass is added 10 lbs. of a phosphopeptide having the following sequence: Glu-Leu-Glu-Glu-Leu-Asn-Val-Pro-Gly-Glu-Ile-Val-Glu-Pse-Leu-Pse-Pse-Pse-Glu-Glu-Ser-Ile-Thr-Arg.

After mixing for a period of 10 minutes at a molten mix temperature of 108°C. of the molten mix is metered from the mixing tank through a positive displacement pump into a laboratory size Readco mixer having counter-rotating mixing blades 2 inches in diameter and a barrel length of 18 inches. The Readco mixer, jacketed with cooling water maintained between 14°-17°C°, is continuously fed at rates of from 15lbs-per-hour at a blade rotation speed of 60 RPM's up to 80 lbs-per-hour at blade speeds of 80 RPM's until the exiting material is sufficiently crystallized such that the product exiting through a 0.5 inch (1.27 cm) orifice into elongated strips is able to maintain its shape. This extrudate is placed onto a water cooled moving stainless steel belt and cut into lengths ranging from 1 to 3 inches. The product coming off the moving cooling belt is placed in storage trays to cool to room temperature in a dry atmosphere for a period of 24 hours, then placed in sealed containers.

### Example 2

### Aliquot blended Crystalline Sorbitol/Phosphopeptide

As in similar Example 1 aqueous sorbitol is cooked and after the water content is reduced to 0.3%, by weight, 10 lbs. of the molten sorbitol is removed and placed into a vessel equipped with a high-shear Sigma blade mixer. This vessel is jacketed such that the temperature of the contents is maintained at 108°C. Then 10 lbs. of phosphopeptide, as describe in Example 1, is mixed into the 10lbs of molten sorbitol until such phosphopeptide is thoroughly dispersed. Then this phosphopeptide/sorbitol mix is reintroduced into the cooker and agitated for 5 minutes.

Then as in Example 1 the mix is pumped into the Readco mixer and formed into 1-3 inches slugs as described.

### Example 3

### Chewing Gum

The crystalline composition of Example 1 is ground in a Waring blender and passed through a 40 mesh screen (U.S. Sieve Series). 54 parts by weight of the screened material is blended with 25 parts gum base; 19.5 parts sorbitol; 0.5 parts glycerine and 1.0 part peppermint flavoring.

## Claims

1. A composition comprising at least one crystalline sugar alcohol selected from the group consisting of sorbitol, mannitol, xylitol, lactitol, cellobiitol and blends of sorbitol/mannitol and sorbitol/xylitol having at least one phosphopeptide or salt thereof, wherein the phosphopeptide comprises the sequence
A-B-C-D-E
wherein A, B, C, D and E are independently phosphoserine, phosphothreonine, phosphotyrosine, phosphohistidine, glutamate and aspartate, uniformly dispersed within its crystal matrix.

2. A composition as claimed in claim 1 wherein said sugar alcohol is sorbitol, xylitol, mannitol or blends thereof.

3. A composition in accordance with claim 1 wherein said sugar alcohol is co-crystallized sorbitol and mannitol.

4. A composition in accordance with claim 3 wherein said co-crystallized sorbitol and mannitol contains between 1 to 30 percent by weight mannitol.

5. A composition in accordance with claim 3 wherein said co-crystallized sorbitol and mannitol contains between 5 to 15 percent by weight mannitol.

6. A composition in accordance with claim 1 wherein said sugar alcohol is co-crystallized sorbitol and xylitol.

7. A composition in accordance with claim 6 wherein said co-crystallized sorbitol and xylitol contains between 5 to 35 percent by weight xylitol.

8. A composition in accordance with claim 6 wherein said co-crystallized sorbitol and xylitol contains between 25 to 30 percent by weight xylitol.

9. A composition in accordance with claim 8 wherein said phosphopeptide is present in amount of 12 percent by weight of the composition.

10. A composition in accordance with claim 1 wherein the phosphopeptide comprises the sequence
A-B-C-D-E
wherein A, B, and C are independently phosphoserine, phosphothreonine, phosphotyrosine and phosphohistidine and D and E are independently phosphoserine, phosphothreonine, glutamate and aspartate.

11. A composition in accordance with claim 1 wherein the phosphopeptide comprises the sequence
A-B-C-D-E
wherein A, B and C are phosphoserine and D and E are glutamate.

12. A composition in accordance with claim 1 wherein said composition is a comestible.

13. A composition in accordance with claim 12 wherein said comestible is a foodstuff or confection.

14. A composition in accordance with claim 12 wherein said composition is in a chewing-gum.

15. A process for producing a crystalline polyol composition having uniformly dispersed within its crystal matrix at least one phosphopeptide compound comprising the steps of:
(A) forming a molten sugar alcohol derived from at least one mono- or polysaccharide;
(B) dispersing particles of the phosphopeptide compound in said molten alcohol to form a homogeneous mixture;
(C) cooling said homogeneous molten mixture while agitating until a viscous mass is formed; and
(D) cooling said mass slowly to a point where said sugar alcohols become fully crystallized.

16. A process in accordance with claim 15 wherein the phosphopeptide compound remains as a discrete particle within the molten alcohol.
